# EUROPEAN PATENT SPECIFICATION

(11) **EP 0 884 057 B1**
(45) Date of publication and mention of the grant of the patent: **20.03.2002**
(21) Application number: 98202100.8
(22) Date of filing: 06.07.1994
(51) Int. Cl.: A01N 1/02, A61L 2/00, A61M 1/14, B01J 19/08

(54) **Apparatus and method for inactivating viral contaminants in body fluids**
Apparatur und Verfahren zur Inaktivierung von viralen Verunreinigungen in Körperflüssigkeit
Appareil et procédé d'inactivation des contaminants viraux des liquides biologiques

(30) Priority: 12.07.1993 US 90381
(43) Date of publication of application: 16.12.1998
(62) Divisional of application: 94923320.9
(73) Proprietor: BAXTER INTERNATIONAL INC., Deerfield, Illinois 60015 (US)
(72) Inventor: Wolf, Ludwig Jr., Illinois 60010 (US); Bratten, William, Illinois 60046 (US); Foley, John, Illinois 60090 (US)
(74) Representative: MacGregor, Gordon

(56) References cited:
- WO-A-90/13334
- WO-A-92/15274
- US-A- 4 726 949
- US-A- 5 030 200

## Description

### BACKGROUND OF THE INVENTION

The present invention relates generally to apparatus and methods for inactivating viral contaminants that may be present in the body fluids. More specifically, the invention relates to apparatus and methods for photodynamically inactivating viral contaminants in body fluids.

In a variety of therapies, such as transfusions and transplants, body fluids, especially blood components such as red blood cells, platelets, plasma, leukocytes, and bone marrow, are collected from one or more individuals and then infused into a patient. Although such therapies provide treatments, some of which are life saving, due to the transmission of infectious diseases there may be potential risks involved with such therapies.

By way of example, it is known that blood can carry infectious agents such as hepatitis virus, human immunodeficiency virus (an etiological agent for AIDS), and herpes virus. Although screening methods exist to identify blood that may include such viruses, blood containing viruses, and other disease causing pathogens, such as bacteria, cannot be 100% eliminated from the pool of possible blood component supplies. Therefore, there is still a small risk that blood transfusions can transmit viral or other infections.

Accordingly, a goal of recent biomedical research has been to reduce the risk of transmitting an infectious agent by selectively inactivating or depleting pathogens present in such blood components. One approach has been to utilize photosensitive (photoactive) agents that when activated by light of the appropriate wavelength will destroy the ability of the pathogen to cause infection. The use of photodynamic therapy has been suggested as a way to eradicate infectious agents from collected blood and its components prior to storage and/or transfusion.

A number of different photoactive agents have been proposed as possibilities to be used to eradicate viral and other contaminants in body fluids. Such photoactive agents include: psoralens; porphyrins; phthalocyanines; and dyes such as methylene blue. See, for example, U.S. Patent Nos. 4,748,120; 4,878,891; 5,120,649; and German Patent Application No. DE 39 30 510 A1 (Mohr).

Although much effort has been focussed on commercializing such methods using photoactive agents, the inventors believe that such methods are currently not commercial. Even though a commercial system for utilizing a photoactive agent to treat blood to eradicate or remove viral and other contaminants has not been developed, it is envisioned that such a system would entail combining the blood with the photoactive agent in a container and irradiating the resultant mixture with light of the appropriate wavelength.

It is known, of course, to use blood pack units to collect blood. The blood pack units include a container typically constructed from a plastic material, usually a polyvinyl chloride material. The blood pack units are connected to tubes that allow blood to be infused into the container as well as to be accessed therefrom.

Of course, blood pack units must be sterilized. Typically, sterilization takes place by steam sterilization at a temperature of above 100°C for a predetermined period of time.

One photoactive agent that appears to be promising with respect to eradicating viruses and bacteria from blood is methylene blue. Methylene blue 3-7-bis(dimethylamino)phenothiazine-5-rum chloride (C₁₆H₁₈CIN₃S), in the presence of light has been reported to damage DNA. Accordingly, it can be used to selectively, in a controlled manner, modify the DNA and RNA of bacterial and viral contaminants thereby inactivating the pathogens. See US Patent No 4,950m655 and EP-A-0574410.

It has previously been found that methylene blue does not migrate into non-PVC material as well as into PVC material under sterilization conditions. Suitable containers providing a steam sterilizable housing for the methylene blue are disclosed in EP-A-0650345. The housing includes at least the surface that contacts the methylene blue solution being constructed from a non-PVC material.

US-A-5,030,200 describes an apparatus and method for externally eradicating contaminants present in body tissues. A photoactive compound, which is preferably a porphyrin or hematoporphyrin, is added to the body tissue and the resulting composition is then irradiated. US-A-5,030,200 also discloses supporting a container holding the body tissue on a transparent support surface and irradiating that container uniformly from both the top and the bottom. The body tissue is illuminated using a light source having a wavelength of from 350 to 1000 nm and preferably from 600 to 700 nm.

WO-90/13334 and US-A-4,726,949 both describe an apparatus and method for irradiating a layer of a blood product containing white blood cells with UV radiation having a wavelength of from 280 to 320 nm. The apparatus which is used comprises a housing, a source of ultraviolet radiation and a means for supporting the blood product layer in the housing. The blood product layer is preferably irradiated from at least two directions.

The present invention provides an apparatus for inactivating viruses in a body fluid with methylene blue according to claim 1.

The invention subjects the container and its contents to a light field under no-flow conditions.

The invention also resides in a method of using the apparatus, as in Claim 10.

In an embodiment, the apparatus includes a container for holding the mixture substantially static within the container during irradiation. The container may contain methylene blue and have a port for infusing the body fluid.

Additional features and advantages of the present invention are described in, and will be apparent from, the detailed description of the presently preferred embodiments and from the drawings.

### BRIEF DESCRIPTION OF THE DRAWINGS

Figure 1 illustrates a perspective view of a device for irradiating a container with a light field.

Figure 2 illustrates a fragmentary cross section of the device of Figure 1.

Figure 3 illustrates a system for irradiating a container with a light field and transporting the container through the light field.

### DETAILED DESCRIPTION OF THE PRESENTLY PREFERRED EMBODIMENTS

The present invention provides apparatus and methods for inactivating pathogens that may be contained in the body fluids. As used herein, body fluid not only includes blood and its components, but also includes other fluids contained in the body such as blood components, leukocytes, red blood cells, white blood cells, plasma (even fresh frozen), etc. or fluid containing structures such as, e.g., bone marrow, semen, and internal organs.

As previously noted, although body fluids, such as blood and its components, can be used in many therapeutic applications, there is the danger of the transfer of infectious disease due to viral and bacterial contaminants that may be contained in such fluids. Recently the use of photoactive agents has provided the hope of inactivating viral and bacterial contaminants that may be contained in such fluids. However, in order to commercialize such methods, certain obstacles must be overcome.

It is known to house blood components in plastic containers. Typically, the plastic container comprises a polyvinyl chloride structure that is plasticized with di(2-ethylhexyl)phthalate (DEHP) and includes stabilizers. Blood components are stored particularly well in such containers. However, it has been found that when a solution of methylene blue, a photoinactive agent of promise, is placed in such a container at a physiological pH of around 7, that upon steam sterilization, the photoinactive agent (methylene blue) migrates into the plastic.

Methods of use of methylene blue to inactivate viral contaminants require rather precise amounts of methylene blue. See, for example, Lambrecht, et al., *Photoinactivation of Viruses in* Human *Fresh Plasma by Phenothiazine Dyes in Combination with Visible Light,* Vox Sang 1991; 60:207-213. Therefore, the migration of the methylene blue solution into the plastic during the sterilization process are believed to provide an unacceptable system.

It has also been found that the migration of methylene blue into the plastic is dependent on a couple of controllable parameters. Therefore, it is possible to provide apparatus and systems wherein methylene blue can be contained within a plastic structure, the structure can be steam sterilized, and the methylene blue solution will be recovered in sufficient quantity to allow the solution to be used to inactivate viral contaminants in a body fluid.

By controlling the type of plastic from which the housing that contains the methylene blue is constructed, the migration of methylene blue into the housing can be controlled.

Of most interest are the more inert plastics, such as polyolefins and polyurethanes. In a preferred embodiment, polypropylene, styrene-ethylene-butylene-styrene (SEBS), ethylenevinyl acetate, and polyesters are used.

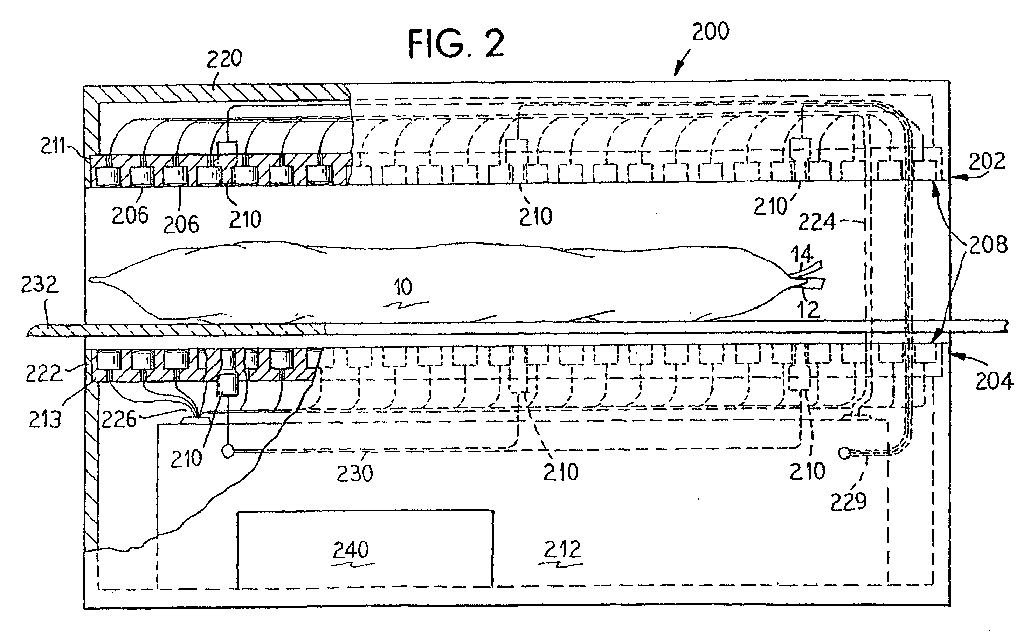

By way of example, referring first to Figure 2, a container 10 is illustrated. The container 10 includes ports 12 and 14 extending therefrom to provide access to an interior of the container 10.

In the container 10 illustrated, the ports 12 and 14 extend from the container 10 and provide means for infusing the body fluid, such as blood or blood components, into the container 10. An example of a system that can be used is the Optipak® system that is disclosed in U.S. Patent No. 4,608,178. In this system, plasma or red blood cells, for example, can be infused into the container 10 through the ports after having been separated from whole blood.

The container 10 can have a structure that is substantially similar to the container for housing blood and blood components available from the Fenwal Division of Baxter International Inc. However, the container 10 can also be constructed so that at least an interior layer that defines the interior surface of the container is constructed from a non-PVC material so as to house methylene blue alone. In that case, most preferably, at least the inner surface of the container 10 is constructed from SEBS, polypropylene, polyester, polyurethane, or ethylenevinyl acetate, or blends thereof. Of course, if desired, the entire container 10 can be constructed from a non-PVC material.

A body fluid such as a blood component and a therapeutically effective amount of methylene blue are mixed together in the container 10. In one embodiment, the methylene blue is added to body fluid already stored in the container 10. In another embodiment, the body fluid is added to a therapeutically effective amount of methylene blue already stored in the container 10. The foregoing can be accomplished by any known sterile means and/or method that has been found to function satisfactorily. In this embodiment, the amount of methylene blue solution used is about 10 ml.

It has been found that the pH of the solution can effect the migration of the solution into the plastic material during sterilization. Preferably, the methylene blue solution has been adjusted to a pH of less than 7.0 and most preferably approximately 6.3 or less.

If methylene blue solution is infused into the container 10 first, the container 10 and methylene blue solution can then be steam sterilized, e.g., at 115°C for 65 minutes. Due to the use of a methylene blue solution having a pH of less than 7.0 and the fact that the container 10 would then include at least an inner surface that is constructed from a non-PVC material, the methylene blue solution, during steam sterilization, will not substantially migrate into the plastic.

A body fluid, such as a blood component, can then be infused into the container 10 through port 14 or 16. To control fluid flow through the port, a breakable cannula or other means can be used.

In the container 10, the body fluid, preferably a blood component, will mix with the methylene blue solution. The container 10 is then irradiated by a light field of the appropriate wavelength (approximately 620-670 nm) to activate the methylene blue within the container 10. This will cause the methylene blue to inactivate any pathogens, e.g., viruses and bacteria, that are contained within the blood component, thus insuring a blood component that does not contain pathogens. The mixture is inactivated for a sufficiently long time and at a suitable wavelength to at least inactivate the viruses in the mixture.

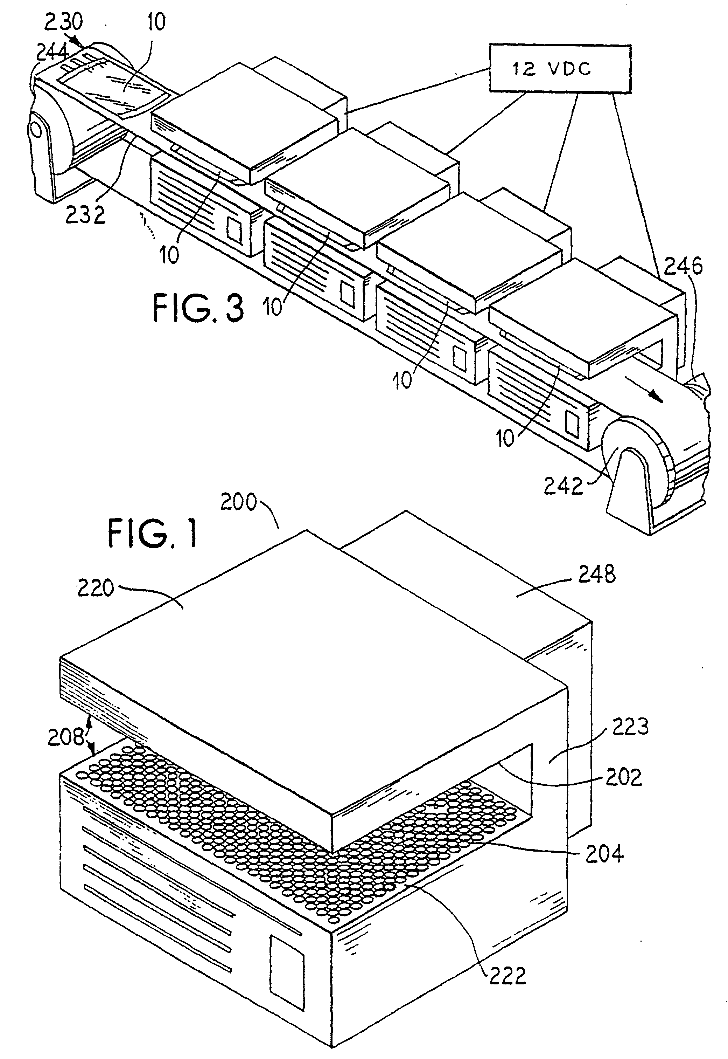

Referring now to both Figures 1 and 2, there is illustrated an apparatus 200 for generating the appropriate light field. As illustrated, the apparatus 200 preferably includes facing arrays 202 *and* 204 of light emitting diodes 206 that generate the light field to which the container 10 can be subjected. As will be appreciated, the apparatus 200 preferably is used for activating methylene blue in single units of fresh frozen plasma.

In an embodiment of the apparatus 200, the light emitting diodes 206 comprise light emitting diodes manufactured by Hewlett Packard under the designation HLMP-8103 and have a light output centered at a wavelength of about 670 nm. Red blood cells essentially are transparent to light radiated at this wavelength. The methylene blue, however, is not. Instead, at this wavelength the methylene blue absorbs the light and becomes activated.

In an embodiment, the light emitting diodes 206 preferably are mounted into printed circuit boards 211 and 213 about the size of 21.5 x 28 cm (8½" x11") sheets of paper. Each board then holds approximately 1280 light emitting diodes forming the relatively large arrays 202 and 204 of light emitting diodes. When powered by a 12 volt DC power supply, the pair 202 and 204 of such arrays can deliver a light field of about 300 mw per centimeter square.

As illustrated, in the apparatus 200, the two arrays 202 and 204 are mounted facing each other in spaced apart relation in such a way that a gap 208 is present within which a container containing about 250 ml of the body fluid, preferably fresh frozen plasma, and containing about 1 µM can be placed between the two arrays 202 and 204. The container can then be irradiated for about 5 minutes, which causes the methylene blue therein to inactivate any virus present.

As a result, the methylene blue and body fluid to be treated can be separately collected and sterilized, and then mixed either in the container in which the methylene blue was collected or the container in which the body fluid was collected. Then, the container can be relatively quickly irradiated by the light field generated by the apparatus 200 so that the mixture therein need not be flowed through flow paths and the like past the sources of light.

In an embodiment, the body fluid and methylene blue are contained in a PVC container. Due to the use of a PVC container, excess methylene, i.e., that methylene blue which is not activated or necessary for photoinactivation of the viruses, leaches out into the container material. Thus, the body fluid can be relatively quickly treated and excess methylene blue is removed from the body fluid.

As illustrated in Figure 2, a device such as the apparatus 200 can be equipped with light sensors 210 that can measure how much light is being delivered to the container 10 and how much light is being transmitted through the container 10 to control a total dosage of light. To this end, the apparatus can include a microprocessor based computer 212 that monitors the sensors 210 and computes cumulative dosages of light. Once a sufficient dosage is reached, the computer 212 can shut off the light emitting diodes 206.

It can be appreciated that some body fluids and/or containers are more opaque than other fluids and/or containers and that therefore the computer 212 must be programmed to accommodate the different light transmissions through the containers due to the differences in opacities of different body fluids.

As illustrated in Figures 1 and 2, in the embodiment illustrated, the apparatus 200 is constructed such that the array 202 is supported within an arm 220 that overhangs a facing support surface 222. The support surface 222 in turn supports or includes the array 204. The overhang 220 is in turn supported by perpendicular wall 223.

Interconnecting lines or cables 224 can be used to interconnect the diodes 206 of the array 202 and the computer 212 while similar lines or cables 226 can be used to interconnect the diodes 206 of the array 204 to the computer 212. In a readily programmable manner, the computer 212 can be used to turn the arrays 202 and 204 off and on as needed.

At the same time, lines or cables 228 can be used to interconnect the sensors 210 in the array 202 with the computer 212 while, similarly, lines or cables 229 can be used to interconnect the sensor 210 in the array 204 with the computer 212.

Referring now to Figure 3, it can be seen that a plurality of such apparatus 200 are placed along a path of travel defined by a conveyor 230 such that a container can be placed on a belt 232 of the conveyor 230 at one end of the conveyor 230 and transported past successive apparatus 200. It can be appreciated that the speed of the belt 232 can be adjusted such that each container 10 placed thereon would be transported past the series of light field apparatus 200 with such a timing (preferably 5 minutes or so) that at the end of the path of travel, the container 10 will have been subjected to a sufficient dosage of light to virally inactivate the body fluid contained therein.

A suitable power supply 240, e.g., a 12 volt DC power supply, can be coupled to each apparatus 200 as illustrated. Moreover, the conveyor 230 can be provided with suitable drive wheels 242 and 244 at opposite ends thereof, as well as a suitably controllable drive motor 246.

It can be appreciated that the belt 232 of the conveyor 230 should be sufficiently translucent to allow both arrays 202 and 204 to generate the appropriate light field. If the belt 232 is not sufficiently translucent, then the light field could be cut up to half and then the exposure time would have to be suitably increased.

Referring to Figure 3, it can be appreciated that all of the apparatus 200 can be connected to a single computer, perhaps even only one of the computers 212 such that the single computer monitors the total dosage of light received by each container 10 passing through the light field. The programming necessary to effect the foregoing is minimal and well within the capabilities of one of ordinary skill in the art, and therefore it is believed to not be necessary to set forth such programming herein.

## Claims

1. An apparatus for inactivating viruses in a body fluid with methylene blue comprising:
light generation means (202, 204), which generates a light field of wavelength and intensity sufficient to activate methylene blue comprising a pair of light sources (202,204) disposed so as to face each other with a gap (208) therebetween,
a support surface (222, 232) located in the gap for supporting a container (10) of body fluid mixed with methylene blue for a time sufficient for viruses to be inactivated on irradiation from the light sources, and
a sensor disposed to sense the amount of light delivered to a container on the support surface (222,232).

2. The apparatus of claim 1, wherein one light source (204) is located beneath the support surface (222, 232) and the other light source (202) is located above the support surface.

3. The apparatus of Claim 1 or 2, including a container (10) for holding the mixture of body fluid and methylene blue in a no-flow state within the container, the container being locatable on the support surface (222, 232) for irradiation.

4. The apparatus of Claim 3, wherein the container (10) contains methylene blue and has a port (12, 14) for receiving a body fluid.

5. The apparatus of Claim 4, wherein the container has at least an inner surface made of a non-polyvinyl chloride material.

6. The apparatus of any preceding claim, wherein each light source comprises an array of light emitting diodes.

7. The apparatus of any preceding claim, wherein each light source (202,204) generates a light field with a wavelength of about 670 nm.

8. The apparatus of any preceding claim, wherein the sensor senses the amount of light transmitted through a container on the support surface (222, 232) and the mixture contained in it.

9. The apparatus of any preceding claim, further comprising a processor coupled to said light sources (202, 204) and sensor and operable to monitor the cumulative amount of light delivered to a container on the support surface (222, 232).

10. A method of using the apparatus of any preceding claim, comprising forming a mixture including methylene blue and a body fluid in a container (10) under sterile conditions and irradiating the container, supported on the support surface (222, 232) for a time sufficient to inactivate viruses in the mixture.

11. The method of claim 10, wherein the body fluid is a blood component.

12. The method of Claim 11, wherein the blood component is selected from the group consisting of plasma, red blood cells, white blood cells, leukocytes, bone marrow and platelets.

13. The method of claim 10, wherein the blood component is fresh frozen plasma.

14. The method of any one of claims 10 to 13, wherein the step of forming the mixture comprises the step of adding the methylene blue to a container (10) in which the body fluid is already stored.

15. The method of Claim 14, as appendant to any one of Claims 1 to 4, or any one of Claims 6 to 9 as appendant to Claims 1 to 4, wherein the body fluid is stored in a container made of polyvinyl chloride material.

16. The method of any one of Claims 10 to 13, wherein the step of forming the mixture comprises the step of adding the body fluid to a container in which the methylene blue is already stored.

17. The method of Claim 10, comprising the further step of allowing excess methylene blue to leach out into the container after the irradiation step.

18. The method of any one of claims 10 to 17, wherein the mixture is irradiated by the light field for a cumulative period of five minutes or greater.

19. The method of any one of claims 10 to 18, including holding the mixture in a no-flow state within the container during radiation.

## Patentansprüche

1. Vorrichtung zum Inaktivieren von Viren in einem Körperfluid mit Methylenblau, wobei die Vorrichtung folgendes aufweist:
- Lichterzeugungseinrichtungen (202, 204), welche ein Lichtfeld mit einer Wellenlänge und einer Intensität erzeugen, die ausreichend sind, um Methylenblau zu aktivieren, und welche ein Paar von Lichtquellen (202, 204) aufweisen, die so angeordnet sind, daß sie einander mit einem Zwischenraum (208) zwischeneinander gegenüberstehen,
- eine Trägerfläche (222, 232), die in dem Zwischenraum angeordnet ist, um einen Behälter (10) für Körperfluid, das mit Methylenblau gemischt ist, für eine Zeitdauer zu tragen, die ausreichend ist, damit Viren bei der Bestrahlung aus den Lichtquellen inaktiviert werden, und
- einen Sensor, der so angeordnet ist, daß er die Lichtmenge mißt, die einem Behälter auf der Trägerfläche (222, 232) zugeführt wird.

2. Vorrichtung nach Anspruch 1,
wobei die eine Lichtquelle (204) unterhalb der Trägerfläche (222, 232) angeordnet ist und die andere Lichtquelle (202) oberhalb der Trägerfläche angeordnet ist.

3. Vorrichtung nach Anspruch 1 oder 2,
die einen Behälter (10) aufweist, um die Mischung aus Körperfluid und Methylenblau in einem nicht-strömenden Zustand innerhalb des Behälters zu halten, wobei der Behälter auf der Trägerfläche (222, 232) zur Bestrahlung positionierbar ist.

4. Vorrichtung nach Anspruch 3,
wobei der Behälter (10) Methylenblau enthält und einen Anschluß (12, 14) besitzt, um ein Körperfluid aufzunehmen.

5. Vorrichtung nach Anspruch 4,
wobei der Behälter zumindest eine Innenoberfläche besitzt, die aus einem Nicht-Polyvinylchlorid-Material besteht.

6. Vorrichtung nach einem der vorhergehenden Ansprüche,
wobei jede Lichtquelle eine Matrix von Licht emittierenden Dioden aufweist.

7. Vorrichtung nach einem der vorhergehenden Ansprüche,
wobei jede Lichtquelle (202, 204) ein Lichtfeld mit einer Wellenlänge von etwa 670 nm erzeugt.

8. Vorrichtung nach einem der vorhergehenden Ansprüche,
wobei der Sensor die Lichtmenge mißt, die durch einen Behälter auf der Trägerfläche (222, 232) und die darin enthaltene Mischung hindurchgeht.

9. Vorrichtung nach einem der vorhergehenden Ansprüche, die ferner einen Prozessor aufweist, der an die Lichtquellen (202, 204) und den Sensor angeschlossen ist und der so betreibbar ist, daß er die kumulative Lichtmenge überwacht, die einem Behälter auf der Trägerfläche (222, 232) zugeführt wird.

10. Verfahren zur Verwendung der Vorrichtung nach einem der vorhergehenden Ansprüche,
das folgende Schritte umfaßt:
- Herstellen einer Mischung, die Methylenblau und ein Körperfluid enthält, in einem Behälter (10) unter sterilen Bedingungen und
- Bestrahlen des Behälters, der auf der Trägerfläche (222, 232) getragen ist, für eine ausreichende Zeitdauer, um Viren in der Mischung zu inaktivieren.

11. Verfahren nach Anspruch 10,
wobei das Körperfluid eine Blutkomponente ist.

12. Verfahren nach Anspruch 11,
wobei die Blutkomponente aus der Gruppe gewählt ist, die aus Plasma, roten Blutzellen, weißen Blutzellen, Leukozyten, Knochenmark und Trombozyten besteht.

13. Verfahren nach Anspruch 10,
wobei die Blutkomponente Fresh-Frozen-Plasma ist.

14. Verfahren nach einem der Ansprüche 10 bis 13,
wobei der Schritt der Herstellung der Mischung den Schritt umfaßt, daß Methylenblau in einen Behälter (10) zugegeben wird, in dem das Körperfluid bereits aufbewahrt wird.

15. Verfahren nach Anspruch 14,
wenn dieses von einem der Ansprüche 1 bis 4 abhängt, oder wenn dieses von einem der Ansprüche 6 bis 9 abhängt, die von den Ansprüchen 1 bis 4 abhängen,
wobei das Körperfluid in einem Behälter aufbewahrt wird, der aus Polyvinylchloridmaterial besteht.

16. Verfahren nach einem der Ansprüche 10 bis 13,
wobei der Schritt der Herstellung der Mischung den Schritt umfaßt, daß das Körperfluid in einen Behälter zugegeben wird, in welchem das Methylenblau bereits aufbewahrt wird.

17. Verfahren nach Anspruch 10,
das den weiteren Schritt aufweist, daß man überschüssiges Methylenblau nach dem Bestrahlungsschritt in das Behältermaterial hinein austreten läßt.

18. Verfahren nach einem der Ansprüche 10 bis 17,
wobei die Mischung von dem Lichtfeld für einen kumulativen Zeitraum von 5 Minuten oder mehr bestrahlt wird.

19. Verfahren nach einem der Ansprüche 10 bis 18,
das den Schritt umfaßt, daß die Mischung innerhalb des Behälters während der Bestrahlung in einem Nicht-Strömungszustand gehalten wird.

## Revendications

1. Appareil pour inactiver des virus dans un liquide biologique avec du bleu de méthylène comprenant :
des moyens de génération de lumière (202, 204) qui génèrent un champ lumineux de longueur d'onde et d'intensité suffisantes pour activer le bleu de méthylène comprenant une paire de sources de lumière (202, 204) disposées de manière à se faire face avec un intervalle (208) entre celles-ci,
une surface de support (222, 232) située dans l'intervalle, destinée à supporter un récipient (10) de liquide biologique mélangé à du bleu de méthylène pendant un intervalle de temps suffisant pour que les virus soient inactivés lors de l'irradiation provenant des sources de lumière, et
un détecteur disposé de manière à détecter la quantité de lumière émise vers un récipient sur la surface de support (222, 232).

2. Appareil selon la revendication 1, dans lequel une source de lumière (204) est située en dessous de la surface de support (222, 232) et l'autre source de lumière (202) est située au-dessus de la surface de support.

3. Appareil selon la revendication 1 ou 2, comprenant un récipient (10) destiné à contenir le mélange de liquide biologique et de bleu de méthylène dans un état de non écoulement à l'intérieur du récipient, le récipient pouvant se situer sur la surface de support (222, 232) en vue de l'irradiation.

4. Appareil selon la revendication 3, dans lequel le récipient (10) contient du bleu de méthylène et comporte un orifice (12, 14) destiné à recevoir un liquide biologique.

5. Appareil selon la revendication 4, dans lequel le récipient présente au moins une surface interne fabriquée à partir de matériau qui n'est pas du polychlorure de vinyle.

6. Appareil selon l'une quelconque des revendications précédentes, dans lequel chaque source de lumière comprend un réseau de diodes électroluminescentes.

7. Appareil selon l'une quelconque des revendications précédentes, dans lequel chaque source de lumière (202, 204) génère un champ lumineux avec une longueur d'onde d'environ 670 mm.

8. Appareil selon l'une quelconque des revendications précédentes, dans lequel le détecteur détecte la quantité de lumière transmise à travers un récipient sur la surface de support (222, 232) et le mélange contenu dans celui-ci.

9. Appareil selon l'une quelconque des revendications précédentes, comprenant en outre un processeur couplé auxdites sources de lumière (202, 204) et au détecteur, et pouvant fonctionner pour contrôler la quantité cumulée de lumière émise vers un récipient sur la surface de support (222, 232).

10. Procédé d'utilisation de l'appareil selon l'une quelconque des revendications précédentes, comprenant la formation d'un mélange comprenant du bleu de méthylène et un liquide biologique dans un récipient (10) dans des conditions de stérilité, et l'irradiation du récipient supporté sur la surface de support (222, 232) pendant un intervalle de temps suffisant pour inactiver les virus dans le mélange.

11. Procédé selon la revendication 10, dans lequel le liquide biologique est un composant sanguin.

12. Procédé selon la revendication 11, dans lequel le composant sanguin est sélectionné parmi le groupe se composant du plasma, des globules rouges, des globules blancs, des leucocytes, de la moelle osseuse et des plaquettes.

13. Procédé selon la revendication 10, dans lequel le composant sanguin est du plasma fraîchement congelé.

14. Procédé selon l'une quelconque des revendications 10 à 13, dans lequel l'étape consistant à former le mélange comprend l'étape consistant à ajouter le bleu de méthylène à un récipient (10) dans lequel le liquide biologique est déjà stocké.

15. Procédé selon la revendication 14, dépendant elle-même de l'une quelconque des revendications 1 à 4, ou de l'une quelconque des revendications 6 à 9 dépendant elles-mêmes des revendications 1 à 4, dans lequel le liquide biologique est stocké dans un récipient fabriqué en polychlorure de vinyle.

16. Procédé selon l'une quelconque des revendications 10 à 13, dans lequel l'étape consistant à former le mélange comprend l'étape consistant à ajouter le liquide biologique à un récipient dans lequel le bleu de méthylène est déjà stocké.

17. Procédé selon la revendication 10, comprenant en outre l'étape consistant à laisser le bleu de méthylène en excès filtrer dans le récipient après l'étape de l'irradiation.

18. Procédé selon l'une quelconque des revendications 10 à 17, dans lequel le mélange est irradié par le champ lumineux pendant un intervalle de temps cumulé de cinq minutes ou davantage.

19. Procédé selon l'une quelconque des revendications 10 à 18, comprenant le fait de maintenir le mélange dans un état de non écoulement à l'intérieur du récipient pendant l'irradiation.
